(19) Europäisches Patentamt
European Patent Office
Office européen des brevets

(11) Numéro de publication: **0 146 439**
**B1**

(12) **FASCICULE DE BREVET EUROPÉEN**

(45) Date de publication du fascicule du brevet: **20.05.87**

(21) Numéro de dépôt: **84402297.0**

(22) Date de dépôt: **13.11.84**

(51) Int. Cl.⁴: **C 07 C 2/00,** C 07 C 29/32, C 07 C 33/048, C 07 C 67/293, C 07 C 69/145, C 07 C 69/738, C 07 C 49/24, C 07 C 43/315

(54) **Procédé de préparation de dérivés acétyléniques, nouveaux dérivés obtenus et leur emploi.**

(30) Priorité: **18.11.83 FR 8318391**

(43) Date de publication de la demande:
**26.06.85 Bulletin 85/26**

(45) Mention de la délivrance du brevet:
**20.05.87 Bulletin 87/21**

(84) Etats contractants désignés:
**AT BE CH DE FR GB IT LI LU NL SE**

(56) Documents cité:
**GB-A-775 723**
**US-A-4 056 573**
**US-A-4 147 672**
**US-A-4 210 610**

**Le dossier contient des informations techniques présentées postérieurement au dépôt de la demande et ne figurant pas dans le présent fascicule.**

(73) Titulaire: **RHONE- POULENC SANTE, Les Miroirs 18 Avenue d'Alsace, F-92400 Courbevoie Cedex (FR)**

(72) Inventeur: **Mignani, Gérard, 2 avenue des Frères Lumière, F-69008 Lyon (FR)**
Inventeur: **Morel, Didier, 3 rue Jean Jaurès, F-91700 Villiers sur Orge (FR)**

(74) Mandataire: **Pilard, Jacques, RHONE- POULENC RECHERCHES Service Brevets Pharma 25, Quai Paul Doumer, F-92408 Courbevoie Cedex (FR)**

**EP 0 146 439 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un nouveau procédé de préparatiom de dérivés acétyléniques de formule générale:

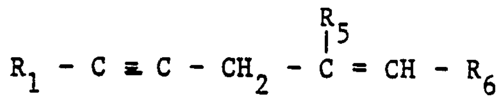

(I)

dans laquelle

- $R_1$ représente un atome d'hydrogène, un radical phényle ou un radical de formule générale:

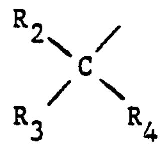

(II)

dans laquelle

$R_2$ et $R_3$, identiques ou différents représentent un atome d'hydrogène ou un radical aliphatique saturé ou non saturé éventuellement substitué par un radical cycloaliphatique à 5 ou 6 chaînons contenant éventuellement une ou deux doubles liaisons et étant éventuellement substitué par un ou plusieurs radicaux alcoyles contenant 1 à 4 atomes de carbone, étant entendu que l'un au moins des symboles $R_2$ et $R_3$ est un radical aliphatique saturé ou non saturé, ou bien $R_2$ et $R_3$ forment ensemble un radical cycloaliphatique à 5 ou 6 chaînons contenant éventuellement une ou deux doubles liaisons et étant éventuellement substitué par un ou plusieurs radicaux alcoyles contenant 1 à 4 atomes de carbone, $R_4$ représente un atome d'hydrogène ou un radical aliphatique saturé ou non saturé ou un radical hydroxy, alcoyloxy dont la partie alcoyle contient 1 à 4 atomes de carbone, alcoylcarbonyloxy dont la partie alcoyle contient 1 à 4 atomes de carbone, méthanesulfonyloxy, benzénesulfonyloxy ou p.toluènesulfonyloxy,

- $R_5$ représente un atome d'hydrogène ou un radical aliphatique saturé, et
- $R_6$ représente un atome d'hydrogène ou un radical aliphatique saturé ou non saturé ou un radical acétyle, formyle éventuellement sous forme d'acétal hydroxy éventuellement sous forme d'éther ou d'ester ou alcoyloxycarbonyle.

Dans ce qui précède et ce qui suit, les radicaux aliphatiques saturés contiennent 1 à 11 atomes de carbone et les radicaux aliphatiques insaturés contiennent 2 à 11 atomes de carbone et une ou plusieurs doubles liaisons et sont éventuellement substitués par un ou plusieurs radicaux, identiques ou différents, choisis parmi les radicaux acétyle ou formyle éventuellement sous forme d'acétal, hydroxy éventuellement sous forme d'éther ou d'ester ou alcoyloxycarbonyle.

Parmi les produits de formule générale (I) ceux pour lesquels $R_1$ est défini comme précédemment, $R_5$ représente un radical aliphatique saturé et $R_6$ représente un radical de formule générale:

- $CH_2CH_2 - R_7$ (III)

dans laquelle $R_7$ représente un atome d'hydrogène ou un radical aliphatique saturé contenant 1 à 9 atomes de carbone ou un radical aliphatique insaturé contenant 2 à 9 atomes de carbone éventuellement substitué par un ou plusieurs radicaux, identiques ou différents, choisis parmi les radicaux acétyle, formyle éventuellement sous forme d'acétal, hydroxy éventuellement sous forme d'éther ou d'ester ou alcoyloxycarbonyle, ou un radical acétyle, formyle éventuellement sous forme d'acétal, un radical hydroxy éventuellement sous forme d'éther ou d'ester ou alcoyloxycarbonyle, sont des produits nouveaux qui constituent un autre objet de la présente invention.

De préférence, les radicaux aliphatiques insaturés ont une structure isoprénique ou polyisoprénique.

D'un intérêt tout particulier sont les produits de formule générale (I) dans laquelle:

- $R_1$ représente un radical de formule générale (II) dans laquelle $R_2$ représente un radical aliphatique saturé contenant 1 à 11 atomes de carbone ou un radical aliphatique insaturé contenant 2 à 11 atomes de carbone et une à trois doubles liaisons, éventuellement substitué par un radical cyclohexyle ou cyclohexényle éventuellement substitué par un ou plusieurs radicaux alcoyles contenant 1 à 4 atomes de carbone,

$R_3$ représente un atome d'hydrogène ou un radical aliphatique sature contenant 1 à 4 atomes de carbone, ou bien

$R_2$ et $R_3$ forment ensemble un radical cyclohexyle ou cyclohexényle éventuellement substitué par un ou plusieurs radicaux alcoyles contenant 1 à 4 atomes de carbone,

$R_4$ représente un atome d'hydrogène ou un radical hydroxy,

- $R_5$ représente un radical aliphatique saturé contenant 1 à 4 atomes de carbone, et
- $R_6$ représente un radical aliphatique saturé contenant 1 à 11 atomes de carbone ou insaturé contenant 2 à 11 atomes de carbone éventuellement substitué par un radical acétyle, formyle éventuellement sous forme d'acétal ou hydroxy sous forme d'éther ou d'ester.

Il est connu, en particulier d'après P. Cadiot et W. Chodkiewicz, Chapitre 9, page 628 et suivantes du livre "The Chemistry of Acetylenes" par H.G. Viehe, Marcel Dekker, New-York (1969), de condenser des dérivés

acétyléniques sur des halogénures allyliques en milieu aqueux en présence d'un sel cuivreux et d'une base, mais cette réaction conduit à un mélange de produits linéaires et ramifiés selon le schéma:

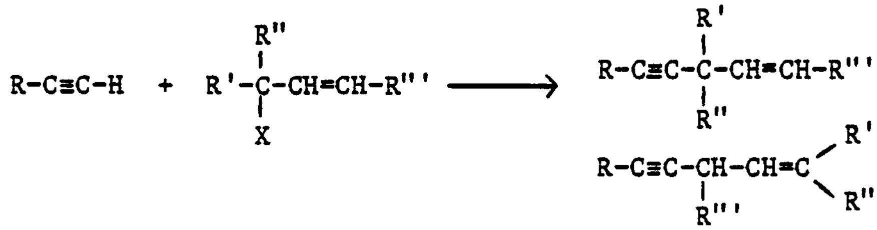

Il est connu également d'après le brevet américain US 4 056 573 de condenser le méthylbutynol sur le chloro-1 acétoxy-4 méthyl-2 butène-2 en présence d'une quantité stoechiométrique d'un sel cuivreux et d'une base organique choisie parmi les amines primaires et secondaires dans un solvant organique tel que le diméthylformamide si l'on opère en milieu non aqueux, ou en présence d'une quantité stoechiométrique de sel cuivreux et d'un agent permettant de tamponner le milieu tel que l'acétate de sodium ou le phosphate de sodium si l'on opère en milieu aqueux. Cependant cette condensation s'effectue avec de mauvais rendements et elle conduit à la formation de produits secondaires indésirables.

Selon la présente invention, les dérivés acétyléniques de formule générale (I) peuvent être obtenus par action d'un dérivé acétylénique de formule générale:

$R_1$- C ≡ C - H (IV)

dans laquelle $R_1$ est défini comme précédemment, sur un dérivé allylique de formule générale:

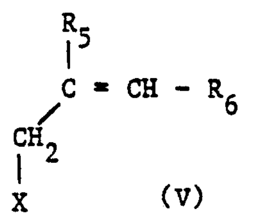

(V) ou

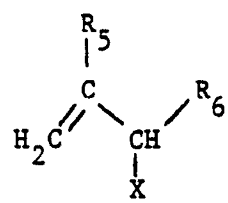

(VI)

dans laquelle $R_5$ et $R_6$ sont définis comme précédemment, et X représente un atome d'halogène ou un radical méthanesulfonyloxy, benzènesulfonyloxy, p.toluènesulfonyloxy ou ammonium quaternaire, en opérant en présence d'un dérivé cuivreux et d'une base organique anhydre.

En opérant dans les conditions de mise en oeuvre du procédé selon l'invention, il se forme essentiellement un produit linéaire quel que soit le dérivé allylique de formule générale (V) ou (VI) utilisé.

Les dérivés du cuivre qui conviennent particulièrement bien sont les dérivés du cuivre (I) ou ceux du cuivre (II) préalablement réduits.

Parmi les dérivés du cuivre (I) peuvent être cités les sels cuivreux tels que CuCl, $Cu_2O$, CuI, CuBr, CuCN, Cu-C≡C-Cu, $CuOCOCH_3$, $CuNO_3$, $C_6H_5$-Cu, $Cu(CH_3COCHCOCH_3)$, $(CH_3)_2C(OH)$-C≡C-Cu et les complexes du cuivre (I) tel que $[CuCl(\text{cyclooctadiène-1,5})]_2$, $CuCl[N(C_2H_5)_3]_3$, $CuCl[N(C_2H_5)_3]_2$, $CuBr[N(C_2H_5)_3]_3$, $[CuCl(NCR)]_n$, $(CuCl\ \text{pyridine})_n$ ou CuCl (adiponitrile).

Parmi les dérivés du cuivre (II) peuvent être cités les sels cuivriques tels que $CuCl_2$, $CuBr_2$, $Cu(CN)_2$, $Cu(OCOCH_3)_2$, $Cu(NO_3)_2$ et les complexes du cuivre (II) tels que $CuCl_2(\text{amine})_n$ ou $CuCl_2(CH_3CN)_2$. Lorsqu'on utilise comme précurseur du catalyseur à base de cuivre (I) un dérivé du cuivre (II), celui-ci est réduit en dérivé du cuivre (I) par le composé acétylénique de formule générale (IV), d'après G. Ellington et W. McCrae, Advances in Organic Chemistry, Vol. 4, p. 225 (1963).

Le catalyseur à base de dérivé du cuivre (I) peut être éventuellement utilisé en présence d'un co-catalyseur choisi parmi les halogénures d'ammonium tels que le chlorure de tétraéthylammonium, les halogénures de phosphonium, tels que le chlorure de tétrabutylphosphonium, ou les halogénures de métal alcalin ou alcalino-terreux. Généralement le rapport molaire co-catalyseur/ catalyseur est compris entre 1 et 50, et de préférence entre 1 et 10.

Généralement, on utilise une quantité de catalyseur à base de dérivé cuivreux représentant de 0,1 à 20 %, et de préférence de 1 à 5 %, en mole du dérivé allylique de formule générale (V) ou (VI).

Comme bases organiques anhydres qui conviennent particulièrement bien peuvent être citées les amines tertiaires telles que la triéthylamine, la tributylamine, la pyridine, la lutidine ou la collidine. Généralement on utilise 1 à 20 moles, et de préférence 1 à 5 moles, de base par mole de dérivé allylique de formule générale (V) ou (VI).

Généralement, le dérivé acétylénique de formule générale (IV) est utilisé à raison de 1 à 20 moles par mole de dérivé allylique de formule générale (V) ou (VI) et de préférence 1 à 4.

La température de réaction est généralement comprise entre 20 et 100° C, mais il est préférable d'opérer entre 40 et 80° C, et la réaction est poursuivie jusqu'à transformation totale du dérivé allylique de formule générale (V) ou (VI).

Il est particulièrement avantageux de mettre en oeuvre le procédé selon la présente invention de telle manière que le catalyseur puisse être recyclé.

En fin de réaction, le mélange réactionnel est constitué essentiellement d'un liquide homogène contenant le produit final de la réaction, les produits de départ n'ayant pas éventuellement réagi, la base organique (triéthylamine) utilisée comme solvant, le catalyseur su cuivre solubilisé et d'un solide précipité constitué essentiellement par le sel de la base organique (chlorhydrate de triéthylamine).

Afin de pouvoir récupérer le complexe du cuivre pour son utilisation dans une opération ultérieure, le mélange réactionnel peut être traité de la manière suivante:

- après séparation de la fraction solide du mélange réactionnel par filtration, le filtrat obtenu est concentré sous pression réduite de manière à obtenir un mélange huileux homogène constitué des produits de la réaction, des produits de départ n'ayant pas éventuellement réagi et de la forme soluble du catalyseur au cuivre,
- le mélange huileux obtenu est traité par un solvant organique ne solubilisant pas les complexes de base organique avec le catalyseur au cuivre tel qu'un hydrocarbure aliphatique (pentane, hexane) ou cycloaliphatique (cyclohexane) ou un éther aliphatique (éther diéthylique) de façon à obtenir un extrait contenant essentiellement le solvant et les produits organiques (produits de la réaction et produits n'ayant pas réagir et de faibles quantités de cuivre et un raffinat contenant essentiellement le complexe catalytique du cuivre et des produits organiques non extraits.

Le traitement par le solvant organique aur le raffinat peut être répété plusieurs fois.

Le raffinat finalement obtenu peut être ainsi utilisé pour la mise en oeuvre d'une opération ultérieure.

Généralement, le taux de récupération du catalyseur est voisin de 90 %.

Les dérivés acétyléniques de formule générale (I) obtenus selon le procédé de la présente invention peuvent être isolés du mélange réactionnel ou des extraits par un solvant approprié et purifiés par application des méthodes habituelles de séparation et de purification utilisées en chimie organique.

Les dérivés acétyléniques de formule générale (I) obtenus selon le procédé de la présente invention sont des intermédiaires particulièrement utiles en synthèse organique et plus spécialement dans la synthèse des vitamines A et E.

D'un intérêt tout particulier sont les produits de formule générale (I) dans laquelle $R_1$ représente un radical de formule générale (II) dans laquelle $R_4$ représente un radical hydroxy, $R_2$ représente un radical méthyle, $R_3$ représente un radical alcoyle contenant 1 à 6 atomes de carbone ou un radical alcényle contenant 3 à 6 atomes de carbone éventuellement substitué par un radical (triméthyl-2,6,6 cyclohexène-1 yl) éthényle ou bien $R_2$ et $R_3$ forment ensemble un radical triméthyl-2,6,6 cyclohexyle, $R_5$ représente un radical méthyle et $R_6$ représente un radical aliphatique saturé contenant 1 à 10 atomes de carbone ou un radical aliphatique insaturé contenant 2 à 10 atomes de carbone et une ou plusieurs doubles liaisons, de préférence deux doubles liaisons en position 1,3, éventuellement substitué par un radical acétyle, formyle éventuellement sous forme d'acétal ou hydroxy éventuellement sous forme d'éther ou d'ester.

Pour l'emploi ultérieur d'un produit de formule générale (I) dans laquelle $R_6$ représente un radical aliphatique insaturé contenant un diène-1,3 terminal, il est possible de fonctionnaliser la molécule dans les conditions décrites dans le brevet européen EP 44 771 par action d'un composé ayant un groupement méthylène activé tel qu'un acétylacétate d'alcoyle.

Par exemple, la condensation du méthyl-3 butynol-3 sur le chloro-3 méthylène-6 méthyl-2 octadiène-1,7 conduit à un alcool acétylénique de formule:

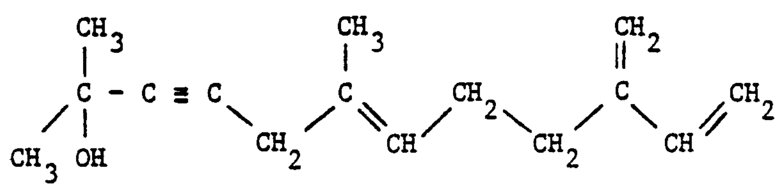

que l'on transforme en phytone, par exemple par action de l'acétylacétate de méthyle dans les conditions décrites dans le brevet européen EP 44 771 suivie de décarboxylation, déshydratation et hydrogénation du produit obtenu. La phytone peut être transformée en vitamine E par application de méthodes connues.

La phytone peut être transformée en vitamine E, en passant intermédiairement par l'isophytol, selon le procédé décrit dans Helv. Chim. Acta, 21, 520-525 et 820-825 (1938).

La vitamine E peut aussi être obtenue par condensation de la triméthylhydroquinone sur le produit de réaction du diméthyl-3,7 hydroxy-3 octène-6 yne-1 sur le chloro-6 méthylène-3 méthyl-7 octadiène-1,7 suivie de la déshydratation et de l'hydrogénation du produit obtenu.

Lorsque dans la formule générale (I), $R_6$ représente un radical saturé ou non saturé substitué par un radical acétyle, formyle éventuellement sous forme d'acétal ou hydroxy sous forme d'éther ou d'ester, ou un radical acétyle, formyle éventuellement sous forme d'acétal, hydroxy sous forme d'éther ou d'ester, il est possible selon la condensation en atomes de carbone des substituants $R_1$, $R_5$ et $R_6$ de préparer la géranylacétone, la phytone ou le citral qui sont des intermédiaires connus pour préparer la vitamine E.

Lorsque dans la formule générale (1), $R_1$ représente un radical de formule générale (II) dans laquelle $R_2$ représente un radical méthyle, $R_3$ représente un radical (triméthyl-2,6,6 cyclo-hexényl-1) éthényl, $R_4$ représente

un radical hydroxy, $R_5$ représente un radical méthyle et $R_6$ représente un radical diméthoxyméthyle, le produit correspondant est particulièrement intéressant pour accéder à la vitamine a après déshydratation et hydrogénation partielle.

Les exemples suivants, donnés à titre non limitatif, montrent comment l'invention peut être mise en pratique.

**EXEMPLE 1**

Dans un ballon en verre de 500 $cm^3$, on introduit successivement, sous atmosphère d'argon, 2 g de iodure cuivreux (10,5 m.moles), 200 $cm^3$ de triéthylamine (1,4 mole), 73,77 g de méthylbutynol $(CH_3)_2C(OH)-C\equiv CH$ (0,877 mole) et 36,84 g de chloro-3 méthylène-6 méthyl-2 octadiène-1,7 (chloro-3 myrcène) titrant 87 % (0,188 mole). Le mélange est agité pendant 28 heures à 50° C.

Après refroidissement, le chlorhydrate de triéthylamine formé est séparé par filtration (29,15 g après séchage). Le dosage par chromatographie en phase gazeuse avec étalon interne de la solution obtenue après filtration (221,77 g) montre que le taux de transformation du chloro-3 myrcène est égal à 99 % et que le rendement en produit de formule:

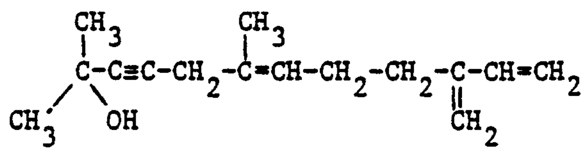

est de 83 %, par rapport au chloro-3 myrcène mis en oeuvre avec une sélectivité de 91 %.

La structure du produit obtenu ($PE_{0,2}$ = 90° C) est confirmée par le spectre de masse et par le spectre de résonance magnétique nucléaire du proton.

**EXEMPLE 2**

Dans un ballon en verre de 50 $cm^3$, on introduit successivement, sous atmosphère d'argon, 94,6 milligrammes de iodure cuivreux (0,5 m.mole), 10 $cm^3$ de triéthylamine (74 m.moles), 3,6 g de méthylbutynol (42,8 m.moles) et 1,56 g de chloro-3 butène-1 (17,2 m.moles). Le mélange est agité pendant 16 heures à 50° C. Après filtration du mélange réactionnel, le dosage de la solution obtenue par chromatographie en phase vapeur montre que le taux de transformation du chloro-3 butène-1 est égal à 100 % et que le rendement en produir de formule:

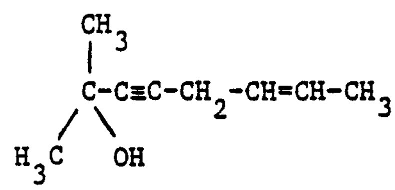

est de 85 % par rapport au chloro-3 butène-1 mis en oeuvre. La structure du produit obtenu est confirmée par le spectre de masse et par le spectre de résonance magnétique nucléaire du proton.

**EXEMPLE 3**

Dans un ballon en verre de 50 $cm^3$, on introduit successivement, sous atmosphère d'argon, 96,8 milligrammes de iodure cuivreux (0,51 m.mole), 10 $cm^3$ de triéthylamine (74 m.moles), 3,63 g de méthylbutynol (43,1 m.moles) et 2,47 g de bromo-1 butène-2 (18,2 m.moles).

Le mélange est agité pendant 16 heures à 50° C. Après filtration du mélange réactionnel, le dosage de la solution obtenue par chromatographie en phase gazeuse montre que le taux de transformation du bromo-1 butène-2 est égal à 100 % et que le rendement en produit de formule:

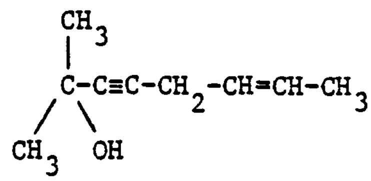

est de 85 % par rapport au bromo-1 butène-2 mis en oeuvre.

**EXEMPLE 4**

Dans un ballon en verre de 50 $cm^3$, on introduit successivement, sous atmosphère d'argon, 95 milligrammes de iodure cuivreux (0,5 m.mole), 10 $cm^3$ de triéthylamine (74 m.moles), 3,54 g de méthylbutynol (42,1 m.moles) et 1,55 g de chlorure de méthallyle (17,1 m.moles).

Le mélange est agité pendant 16 heures à 50°C. Après filtration du mélange réactionnel, le dosage de la solution obtenue par chromatographie en phase vapeur montre que le taux de transformation du chlorure de méthallyle est égal à 100 % et que le rendement en produit de formule:

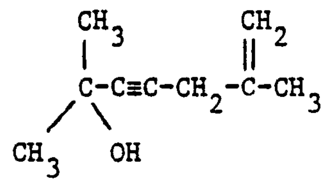

est égal à 100 % par rapport au chlorure de méthallyle mis en oeuvre.

La structure du produit obtenu est confirmée par le spectre de masse, le spectre infra-rouge et le spectre de résonance magnétique nucléaire du proton.

**EXEMPLE 5**

Dans un ballon en verre de 50 $cm^3$, on introduit successivement, sous atmopshère d'argon, 0,1 g de iodure cuivreux (0,52 m. mole), 20 $cm^3$ de triéthylamine (148 m.moles), 5,04 g de méthylbutynol (60 m.moles) et 3,25 g de chloro-1 méthyl-2 acétoxy-4 butène-2 (20 m.moles).

Le mélange est agité pendant 12 heures à 60°C. Il y a formation d'un précipité blanc de chlorhydrate de triéthylamine qui est séparé par filtration. Le mélange réactionnel est lavé à l'eau puis après décantation on extrait la phase aqueuse par 3 fois 20 $cm^3$ d'acétate d'éthyle. Les phases organiques réunies sont séchées sur sulfate de magnésium. Après filtration et évaporation du solvant on obtient 7,7 g d'une huile jaune dont l'analyse par chromatographie en phase gazeuse montre la présence de 54 % du produit de formule:

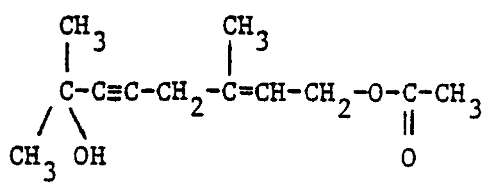

Le rendement est de 98 % par rapport au chloro-1 méthyl-2 acétoxy-4 butène-2 mis en oeuvre.

La structure du produit obtenu est confirmée par le spectre de masse et par le spectre de résonance magnétique nucléaire du proton.

**EXEMPLE 6**

Dans un ballon en verre de 250 $cm^3$, on introduit successivement, sous atmosphère d'argon, 0,5 g de iodure cuivreux (2,6 m.moles), 20 $cm^3$ de triéthylamine (148 m.moles), 10 $cm^3$ de méthylbutynol (103 m.moles), 1,49 g de chloro-1 myrcène (chloro-1 méthyl-2 méthylène-6 octadiène-2,7) (8,7 m.moles) et 1 g de chloro-3 myrcène (5,8 m.moles).

Le mélange est agité pendant 48 heures à 50°C. Après filtration du mélange réactionnel, le dosage de la

solution obtenue par chromatographie en phase gazeuse montre que le taux de transformation du mélange chloro-1 myrcène et chloro-3 myrcène est égal à 97 % et que le rendement en produit obtenu, qui est identique à celui obtenu dans l'exemple 1, est de 66 % par rapport aux chloromyrcènes mis en oeuvre. La sélectivité est de 81 %.

**EXEMPLE 7**

Dans un ballon en verre de 50 $cm^3$, on introduit successivement, en atmosphère d'argon, 95,3 mg de iodure cuivreux (0,5 m. mole), 1,83 g de triéthylamine (18 m.moles), 10 $cm^3$ de méthylbutynol (103 m.moles) et 2,60 g de chloro-1 méthyl-2 diméthoxy-4,4 butène-2 (15,8 m.moles).

Le mélange est agité pendant 16 heures à 60°C. Après filtration du mélange réactionnel et élimination des produits volatils, on obtient 6,22 g d'huile dont le dosage par chromatographie en phase vapeur montre que le taux de conversion du chloro-1 diméthoxy-4,4 méthyl-2 butène-2 est égal à 68 % et que le rendement en produit de formule:

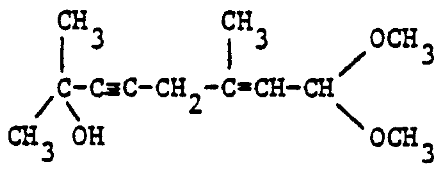

est de 60 % par rapport au chloro-1 diméthoxy-4,4 méthyl-2 butène mis en oeuvre. La sélectivité est de 95 %.

**EXEMPLE 8**

Dans un ballon en verre de 250 $cm^3$, on introduit successivement, sous atmosphère d'argon, 0,2 g de iodure cuivreux (1 m.mole), 30 $cm^3$ de triéthylamine (0,22 mole), 7 $cm^3$ de méthylbutynol (72,3 m.moles) et 11 g d'un mélange contenant 5,5 g de géranylacétone et 5,5 g de chloro-3 géranylacétone (chloro-3 diméthyl-2,6 oxo-10 undécadiène-1,6) (24 m.moles).

Le mélange est agité pendant 16 heures à 50°C. Après filtration du mélange réactionnel, le dosage par chromatographie en phase gazeuse montre que le taux de transformation de la chloro-3 géranylacétone est égal à 100 % et que le rendement en produit de formule:

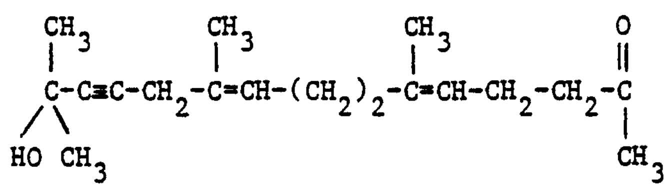

est de 85 % par rapport à la chloro-3 géranylacétone mise en oeuvre.

La structure du produit obtenu est confirmée par le spectre de résonance magnétique nucléaire du proton, le spectre de masse et le spectre infra-rouge.

**EXEMPLE 9**

Dans un ballon en verre de 50 $cm^3$, on introduit successivement, sous atmosphère d'argon, 19 mg de iodure cuivreux (0,1 m.mole), 10 $cm^3$ de triéthylamine (74 m.moles), 3,06 g de phénylacétylène (30 m.moles) et 1,70 g de chloro-3 myrcène (10 m.moles).

Le mélange est agité pendant 8 heures à 80°C. Après filtration du mélange réactionnel, le dosage par chromatographie en phase vapeur montre que le taux de transformation du chloro-3 myrcène est égal à 100 % et que le rendement en produit de formule:

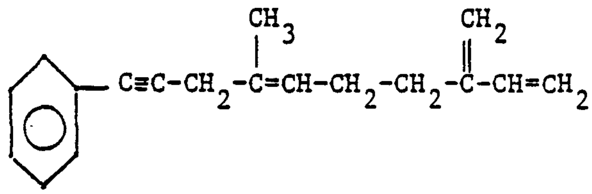

est de 72 % par rapport au chloro-3 myrcène mis en oeuvre. La sélectivité est de 87 %.
La structure du produit obtenu est confirmée par le spectre de résonance magnétique nucléaire du proton

**EXEMPLE 10**

Dans un ballon en verre de 100 $cm^3$, on introduit successivement, sous atmosphère d'argon, 10 mg de chlorure cuivreux (0,1 m.mole), 0,1 g de iodure de potassium (0,6 m.mole), 20 $cm^3$ de triéthylamine (148 m.moles), 5,04 g de méthylbutynol (60 m.moles) et 3,41 g de chloro-3 myrcène (20 m.moles).

Le mélange est agité pendant 16 heures à 60°C. Après filtration du mélange réactionnel, le dosage par chromatographie en phase gazeuse montre que le taux de transformation du chloro-3 myrcène est égal à 98,7 % et que le rendement en produit identique à celui obtenu à l'exemple 1 est égal à 68 % par rapport au chloro-3 myrcène engagé.

La sélectivité est de 87 %.

**EXEMPLE 11**

Dans un ballon de 100 $cm^3$, on introduit successivement, sous atmosphère d'argon, 0,5 g de iodure cuivreux (2,6 m.moles), 40 $cm^3$ de triéthylamine (296 m.moles), 15,3 g de déhydrolinalol [$(CH_3)_2C=CH-CH_2CH_2-C(CH_3)(OH)-C\equiv CH$] (0,1 mole) et 8,52 g de chloro-3 myrcène (50 m.moles).

Le mélange est agité pendant 72 heures à 50°C.

On observe la formation d'un précipité blanc de chlorhydrate de triéthylamine. Après filtration, on obtient 17 g d'une huile dont le dosage par chromatographie en phase gazeuse montre que le taux de transformation du déhydrolinalol est égal à 32 % et que le rendement en produit de formule:

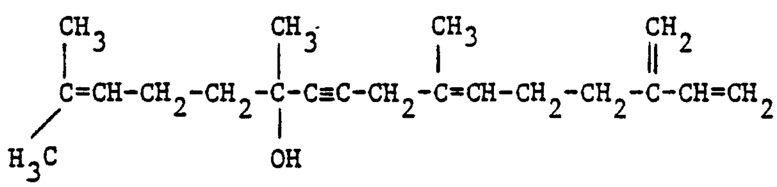

est de 31,4 % par rapport au déhydrolinalol mis en oeuvre.
La sélectivité est de 85 %.
La structure du produit obtenu est confirmée par le spectre infra-rouge et par le spectre de résonance magnétique nucléaire du proton.

**EXEMPLE 12**

Dans un ballon en verre de 100 $cm^3$, on introduit successivement, sous atmosphère d'argon, 0,134 g de chlorure cuivrique anhydre (1 m.mole), 30 $cm^3$ de triéthylamine (222 m.moles), 5,04 g de méthylbutynol (60 m.moles) et 3,40 g de chloro-3 myrcène (20 m.moles).

Le mélange est agité pendant 64 heures à 50°C. Après élimination du chlorhydrate de triéthylamine par filtration et concentration sous pression réduite, on isole 0,92 g (0,55 m.mole) d'un produit de formule:

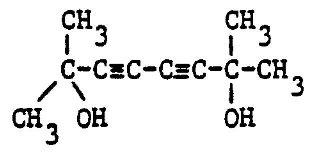

formé lors de la réduction du chlorure cuivrique en chlorure cuivreux catalysant la réaction.

L'analyse du mélange réactionnel par chromatographie en phase gazeuse montre que le taux de transformation du chloro-3 myrcène est égal à 77 % et le rendement en produit identique à celui obtenu dans l'exemple 1 est égal à 65,7 % par rapport au chloro-3 myrcène engagé.

La sélectivité est de 85,3 %.

**EXEMPLE 13**

Dans un ballon en verre de 100 $cm^3$, on introduit successivement, sous atmosphère d'argon, 0,5 g de iodure cuivreux (2,62 m.moles), 60 $cm^3$ de triéthylamine (0,44 mole), 16,6 g de éthynyl-1 hydroxy-1 triméthyl-2,2,6 cyclohexane (0,1 mole) et 8,52 g de chloro-3 myrcène (50 m.moles).

Le mélange est agité pendant 48 heures à 50°C. Après filtration du mélange réactionnel, le dosage par chromatographie en phase gazeuse montre que le rendement en produit de formule:

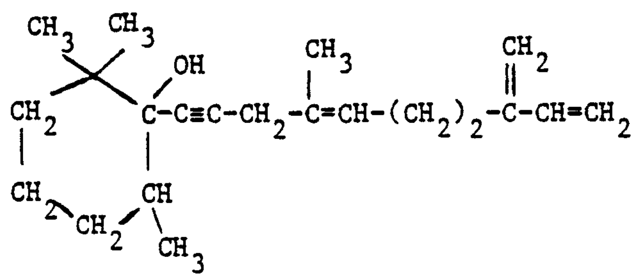

est de 33 % par rapport au chloro-3 myrcène mis en oeuvre.

La sélectivité est de 95 %.

La structure du produit obtenu est confirmée par le spectre de masse, le spectre infra-rouge et le spectre de résonance magnétique nucléaire du proton.

**EXEMPLE 14**

Dans un ballon en verre de 250 $cm^3$, on introduit successivement, sous atmosphère d'argon, 0,4 g de iodure cuivreux (2,1 m.moles), 30 $cm^3$ de triéthylamine (222 m.moles), 4,7 g d'éthynyl β-ionol (21,5 m.moles) et 21,16 g de chlorure de méthallyle (0,23 mole).

Le mélange est agité pendant 40 heures à 50°C. Après filtration du mélange réactionnel, le dosage par chromatographie en phase gazeuse montre que le taux de transformation de l'éthynyl β-ionol est égal à 83 % et que le rendement en produit de formule:

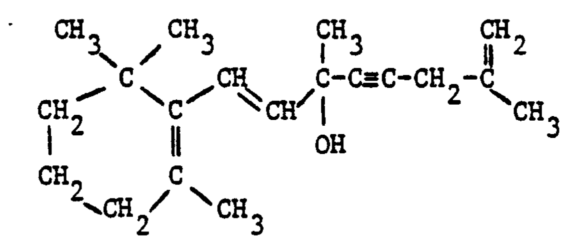

est de 56,7 % par rapport à l'éthynyl β-ionol mis en oeuvre.

La structure du produit obtenu est confirmée par le spectre de résonance magnétique nucléaire du proton.

## EXEMPLE 15

Dans un ballon en verre de 160 $cm^3$, on introduit successivement, sous atmosphère d'argon, 0,445 g de $[CuCl(cyclooctadiène\text{-}1,5)]_2$ soit 2,18 milliatome-gramme de Cu, 40 $cm^3$ de triéthylamine (285 m.moles) et 7,90 g de chloro-3 myrcène titrant 83 % (38,6 m. moles). On chauffe le mélange à 60°C en 1 heure et on ajoute alors 15,02 g de méthylbutynol (192 m.moles). On maintient la température à 60°C, sous agitation, pendant 6 heures 30 minutes. On observe la formation d'un précipité blanc de chlorhydrate de triéthylamine qui est éliminé par filtration.

Le dosage par chromatographie en phase gazeuse montre que le taux de conversion du chloro-3 myrcène est égal à 99,7 % et que le rendement en produit identique à celui obtenu à l'exemple 1 est de 79,4 % par rapport au chloro-3 myrcène mis en oeuvre. La sélectivité est de 88 %.

## EXEMPLE 16

Dans un réacteur en verre de 160 $cm^3$ équipé de chicanes en acier inoxydable et d'une turbine assurant l'agitation, on introduit successivement, sous atmosphère d'argon, 83,4 milligrammes de $[RhCl(cyclooctadiène\text{-}1,5)]_2$ (0,34 milliatome-g de rhodium), 2,12 g de sel de sodium de triphénylphosphine méta trisulfonée soit 3,4 milliatome-g de $P^{+3}$, 0,161 g de $Na_2CO_3$, 34 $cm^3$ d'eau distillée, 4 $cm^3$ de méthanol, 26,82 g d'acétylacétate de méthyle (0,231 mole) et 32,97 g du produit obtenu à l'exemple 1 (0,151 mole).

Le mélange réactionnel est agité pendant 6 heures à 80°C. Après refroidissement et décantation, on récupère 51,14 g de liquide contenant 46,92 g d'un mélange équimolaire des produits de formule:

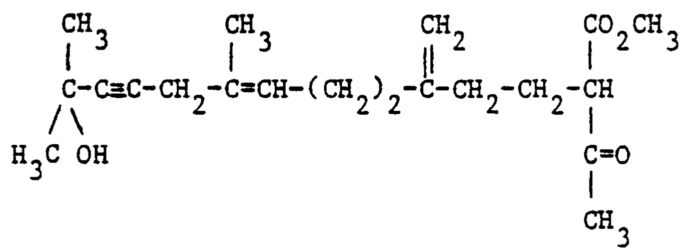

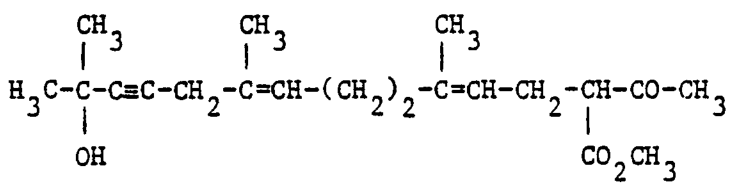

Le taux de transformation du produit obtenu à l'exemple 1 est égal à 98,7 % et celui de l'acétylacétate de méthyle est égal à 64 %.

Le rendement par rapport au produit obtenu à 1 exemple mis en oeuvre est égal à 93 %. La sélectivité est de 94,3 %.

Dans un ballon en verre de 500 $cm^3$ muni d'un barreau aimanté, on introduit, sous atmosphère d'argon, 43,65 g du mélange obtenu précédemment titrant 91,4 % (0,12 mole), 100 $cm^3$ d'eau et 17,2 g de lessive de soude à 30,75 % (0,132 mole). On maintient l'agitation pendant 16 heures à 20°C. On ajoute alors 7,11 g d'acide sulfurique dilué dans 25 $cm^3$ d'eau. La décarboxylation s'opère dès 20°C et il se forme une couche organique que l'on sépare par décantation. On récupère 33 g d'un mélange des produits de formule:

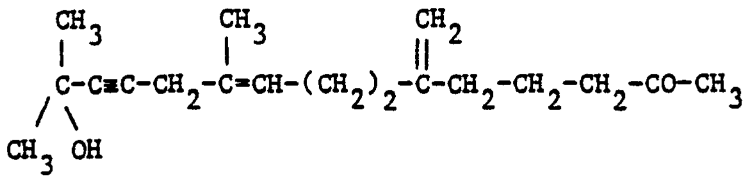

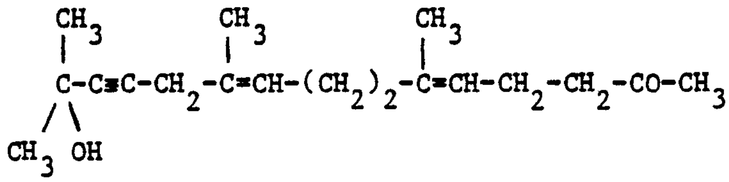

Le rendement est de 99 %.

Dans un ballon en verre de 100 $cm^3$ muni d'un dispositif permettant de distiller, on introduit, sous atmosphère d'argon, 12,03 g du mélange obtenu précédemment (43,6 m.moles), 50 $cm^3$ de toluène et 0,11 g d'acide sulfurique. On chauffe à reflux pendant 30 minutes en éliminant l'eau qui se forme lors de la déshydratation. Après refroidissement, on introduit 0,2 g de bicarbonate de sodium (1,9 m.mole) et on laisse réagir pendant 16 heures. La solution toluénique est ensuite filtrée et introduite dans un autoclave en acier inoxydable contenant 0,5 g de Pd/C à 10 %. On chauffe à 50°C et on maintient sous une pression de 3 bars d'hydrogène pendant 8 heures.

Après élimination du catalyseur par filtration et évaporation du solvant, on récupère 11,5 g de liquide titrant 98 % en phytone. Le rendement est égal à 96,5 % par rapport au produit déshydraté mis en oeuvre.

La structure de la phytone est confirmée par le spectre de résonance magnétique nucléaire $^{13}C$ et par le spectre de masse.

**EXEMPLE 17**

Dans un ballon en verre de 50 $cm^3$, on introduit successivement, sous atmosphère d'argon, 97,4 mg de iodure cuivreux (0,51 m.mole), 10 $cm^3$ de triéthylamine (73,6 m.moles), 5,30 g d'acétate de méthylbutynyle

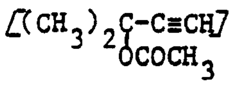

(42 m.moles) et 3 g de chloro-3 myrcène titrant 83,4 % (14,7 m.moles)

Le mélange réactionnel est agité pendant 48 heures à 50°C. Après élimination par filtration du chlorhydrate de triéthylamine et traitement à l'eau, on récupère 3,94 g de liquide contenant le produit de formule:

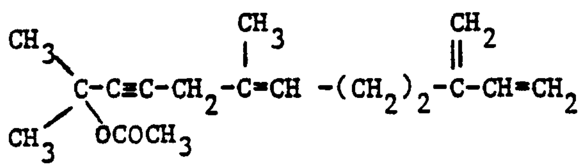

**EXEMPLE 18**

Dans un ballon en verre de 50 $cm^3$, on introduit successivement, sous atmosphère d'argon, 95,9 mg de iodure cuivreux (0,50 m.mole), 10 $cm^3$ de triéthylamine (73,4 m.moles), 3,6 g de méthylbutynol (42,8 m.moles) et 3,5 g d'un mélange équinolaire de méthyl-2 chloro-3 méthylène-6 oxo-10 undécène-1 et de chloro-3 géranylacétone (15,3 m.moles).

Le mélange est agité pendant 72 heures à 60°C. Après élimination par filtration du chlorhydrate de triéthylamine formé et traitement à l'eau, on récupère un mélange contenant 3,11 g de produits de formule:

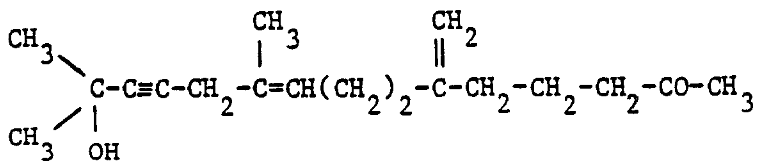

et

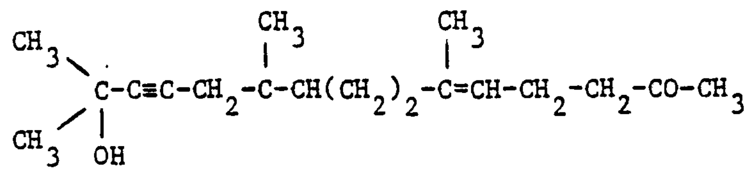

Le rendement est de 73,6 %.

**EXEMPLE 19**

Dans un réacteur en verre de 160 cm$^3$ à agitation centrale assurée par une turbine, on introduit successivement, sous atmosphère d'argon, 0,401 g de iodure cuivreux (2,1 m.moles), 40 cm$^3$ de triéthylamine (0,285 mole), 7,74 g de chloro-3 myrcène titrant 83,4 % (37,9 m.moles). Le mélange réactionnel est chauffé à 60° C sous agitation puis on ajoute, en 5 minutes, 15,02 g de méthylbutynol (178 m.moles).

Le mélange est maintenu pendant 23 heures à 60° C.

Le taux de transformation du chloro-3 myrcène est égal à 100 % et le rendement en produit identique à celui obtenu à l'exemple 1 est de 81,5 % par rapport au chloro-3 myrcène mis en oeuvre.

La sélectivité est de 87 %.

**EXEMPLE 20**

Dans un réacteur en verre de 1,3 litre à agitation centrale assurée par une turbine, on introduit successivement, sous atmosphère d'argon, 4,0116 g de chlorure cuivreux (40,5 m.moles), 740 cm$^3$ de triéthylamine (5,284 moles) et 183,38 g de chloro-3 myrcène titrant 84,8 % (0,914 mole). On porte le mélange à 53,5° C sous agitation puis on ajoute, en 30 minutes, 255,31 g de méthylbutynol (3,035 moles).

Le mélange est maintenu pendant 22 heures à 60° C.

Le taux de transformation du chloro-3 myrcène est égal à 100 % et le rendement en produit identique à celui obtenu à l'exemple 1 est de 81 % par rapport au chloro-3 myrcène engagé.

La sélectivité est de 87 %.

**EXEMPLE 21**

Dans un ballon en verre de 250 cm$^3$, on introduit successivement, sous atmosphère d'argon, 0,15 g de $Cu_2O$ (1,04 m.mole), 40 cm$^3$ de triéthylamine (0,285 mole) et 7,43 g de chloro-3 myrcène titrant 83,4 % (36,3 m.moles).

On porte le mélange à 60°C pendant 30 minutes sous agitation puis on ajoute en 5 minutes, 14,81 g de méthylbutynol (0,176 mole). Le mélange est maintenu pendant 9 heures à 60°C.

Le taux de transformation du chloro-3 myrcène est égal à 98,1 % et le rendement en produit identique à celui obtenu à l'exemple 1 est de 74,7 % par rapport au chloro-3 myrcène mis en oeuvre.

La sélectivité est de 89,2 %.

**EXEMPLE 22**

Dans un réacteur en verre de 0,4 litre à agitation centrale assurée par une turbine, on introduit successivement, sous atmosphère d'argon, 2,11 g de chlorure cuivreux (21,3 m.moles), 200 cm$^3$ de triéthylamine (1,39 mole) et 15,45 g de chloro-3 myrcène titrant 95 % (0,42 mole).

On porte le mélange à 60°C sous agitation, puis on coule en 10 minutes 37,47 g de méthylbutynol (0,445 mole). Le mélange est maintenu pendant 9 heures à 60°C.

Après élimination du chlorhydrate de triéthylamine formé par filtration et lavage du précipité avec la triéthylamine, on récupère 193,28 g de solution organique.

Le taux de transformation du chloro-3 myrcène est égal à 92,6 % et le rendement en produit identique à celui obtenu à l'exemple 1 est de 80,2 % par rapport au chloro-3 myrcène engagé.

La sélectivité est de 87,1 %.

**EXEMPLE 23**

Dans un ballon en verre de 250 $cm^3$, on introduit successivement sous atmosphère d'argon, 0,788 g de iodure cuivreux (4,1 m.moles), 50 $cm^3$ de triéthylamine (368 m.moles), 21,96 g d'un mélange 35-65 de chloro-3 diméthyl-2,6 carbométhoxy-9 undécadiène-1,6 one-10 et de chloro-3 méthyl-2 méthylène-6 carbométhoxy-9 undécène-1 one-10 (76,6 m.moles) et 14,02 g de méthylbutynol (167 m.moles). Le mélange réactionnel est agité à 60°C pendant 20 heures. Après filtration du mélange réactionnel pour éliminer le chlorhydrate de triéthylamine et concentration, on récupère 30,12 g d'une huile contenant un mélange des produits de formule:

$$(CH_3)_2C(OH)-C\equiv C-CH_2-C(CH_3)=CH-(CH_2)_2-\overset{\overset{CH_2}{\|}}{C}-CH_2CH_2-CH(COCH_3)COOCH_3 \quad \text{et}$$

$$(CH_3)_2C(OH)-C\equiv C-CH_2-C(CH_3)=CH-(CH_2)_2-\overset{\overset{CH_3}{|}}{C}=CH-CH_2-CH(COCH_3)COOCH_3$$

Le taux de transformation des produits mis en oeuvre est égal à 100 %.

**EXEMPLE 24**

Dans un réacteur en verre de 400 $cm^3$ muni d'une agitation centrale assurée par une turbine, on introduit, sous atmosphère d'argon, 1,98 g de chlorure cuivreux (20 m.moles), 1,495 g de chlorure de potassium (20 m.moles), 200 $cm^3$ de triéthylamine (1,39 moles) et 76,37 g de chloro-3 méthylène-6 méthyl-2 octadiène-1,7 (chloro-3 myrcène) titrant 92 % soit 0,413 mole.

Le mélange est chauffé à 60° C sous agitation, puis on ajoute, en 10 minutes, 37,52 g de méthylbutynol (0,446 mole). Le mélange est maintenu à 60° C pendant 18 heures.

Après refroidissement à 20° C, le chlorhydrate de triéthylamine formé est séparé par filtration et est lavé par 150 $cm^3$ d'acétone. Le précipité sec pèse 39,7 g et il contient 3,6 mg de cuivre.

Le filtrat obtenu pèse 266,88 g.

L'analyse de ce filtrat montre que le taux de transformation du chloro-3 myrcène est de 100 % et que le rendement en produit de formule:

$$CH_3-\underset{\underset{OH}{|}}{\overset{\overset{CH_3}{|}}{C}}-C\equiv C-CH_2-\overset{\overset{CH_3}{|}}{C}=CH-CH_2CH_2-\overset{\overset{CH_3}{|}}{C}-CH=CH_2 \quad (A)$$

est de 69,4 % par rapport au chloro-3 myrcène mis en oeuvre. La sélectivité est de 88 %.

Le filtrat est concentré sous pression réduite (20 mm de mercure; 2,7 kPa) à 40°C. On obtient sinsi 115,5 g d'une huile rouge dont l'analyse montre qu'elle contient 1,270 g de cuivre (1,1 %) et 62,1 g de produit de condensation (A).

Cette huile est agitée pendant 30 minutes avec 500 $cm^3$ d'hexane puis on laisse décanter pendant 1 heure.

On obtient ainsi un extrait hexanique (336,13 g) contenant 34,06 g du produit de condensation (A) et 0,144 g de cuivre.

Le raffinat est traité par 250 $cm^3$ d'hexane pendant 30 minutes puis on laisse décanter pendant 1 heure.

On obtient un extrait hexanique (181,6 g) contenant 9,28 g de produit de condensation (A) et 0,009 g de cuivre.

Le raffinat est traité par 250 $cm^3$ d'hexane pendant 30 minutes puis on laisse décanter pendant 1 heure.

On obtient un extrait hexanique (170,22 g) contenant 5,37 g de produit de condensation (A) et ne contenant pas de cuivre.

Le raffinat est traité à nouveau par 250 $cm^3$ d'hexane pendant 30 minutes puis on laisse décanter pendant 1 heure.

On obtient ainsi un extrait hexanique (162,57 g) contenant 4,1 g de produit de condensation (A).

Le raffinat finalement obtenu (34,65 g), qui contient 7,84 g de produit de condensation (A) et 1,019 g de cuivre, peut être utilisé dans une opération ultérieure de condensation.

On récupère ainsi 80 % du cuivre mis en oeuvre.

Par concentration des extraits hexaniques, on obtient 52,81 g de produit de condensation (A).

**EXEMPLE 25**

Dans un réacteur en verre de 160 $cm^3$ muni d'une agitation centrale assurée par une turbine, on introduit successivement, sous atmosphère d'argon, 0,203 g de chlorure cuivreux (2 m.moles), 20 $cm^3$ de triéthylamine (145 m.moles) et 12,14 g d'un mélange 45/55 de chloro-3 diméthyl-2,6 carbométhoxy-9 undécadiène-1,6 one-10 et de chloro-3 méthyl-2 méthylène-6 carbométhoxy-9 undécène-1 one-10 titrant 85 % en poids, soit 36 m.moles.

On chauffe le mélange réactionnel à 60° C sous agitation, puis on ajoute, en 1 minute, 3,72 g de méthylbutynol (44,2 m.moles). Le mélange réactionnel est maintenu à 60° C pendant 23 heures.

Après refroidissement à 20° C, le chlorhydrate de triéthylamine est séparé par filtration et est lavé avec 30 $cm^3$ d'acétone.

Le précipité sec pèse 3,91 g et contient 0,72 mg de cuivre.

Le filtrat obtenu pèse 57,86 g.

L'analyse du filtrat montre que le taux de transformation des produits chlorés mis en oeuvre est de 100 % et que le rendement en produits de condensation constitué d'un mélange 45/55 de:

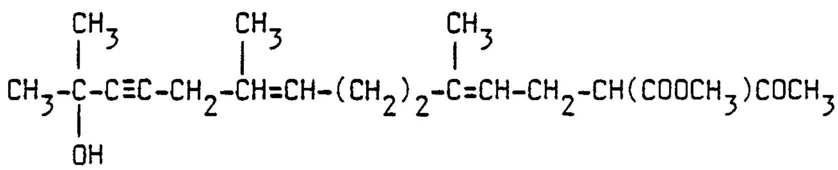

et

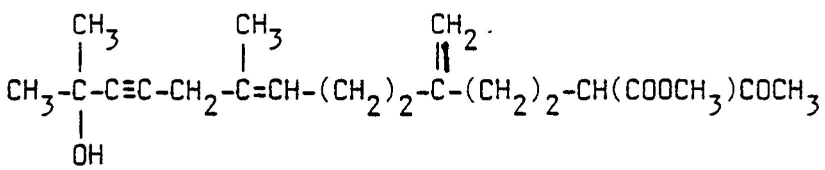

est de 78,9 % par rapport aux produits chlorés mis en oeuvre. La sélectivité est de 95 %.

Le filtrat est concentré sous pression réduite (20 mm de mercure; 2,7 kPa) à 40° C. On obtient ainsi 15,54 g d'une huile rouge contenant 9,5 g des produits de condensation et 0,124 g de cuivre.

L'huile est extraite 6 fois successives par de l'éther éthylique après décantation pendant 1 heure à chaque fois.

Les résultats des extractions successives sont rassemblés dans le tableau suivant:

| N° de l'extraction | Ether éthylique (cm3) | Durée de l'agitation (heure) | Composition de l'extrait éthéré après concentration | |
|---|---|---|---|---|
| | | | Produit (g) | Cuivre (g) |
| 1 | 60 | 3/4 | 8,22 | 0,0015 |
| 2 | 50 | 1/2 | 0,79 | |
| 3 | 40 | 1 1/2 | 0,46 | |
| 4 | 40 | 1 1/2 | 0,1 | |
| 5 | 40 | 2 | 0 | |
| 6 | 40 | 2 | 0,05 | |

Le raffinat finalement obtenu, qui contient 5,11 g de produits de condensation et 0,114 g de cuivre, peut être utilisé dans une opération ultérieure de condensation.

On récupère ainsi 89,7 % de cuivre mis en oeuvre.

**EXEMPLE 26**

Dans un réacteur en verre de 160 $cm^3$ muni d'une agitation centrale assurée par une turbine, on introduit successivement, sous atmosphère d'argon, 0,8 g de chlorure cuivreux (8,08 m.moles), 80 $cm^3$ de triéthylamine (0,58 mole) et 45,90 g d'un mélange 45/55 de chloro-3 diméthyl-2,6 carbométhoxy-9 undécadiène-1,6 one-10 et de chloro-3 méthyl-2 méthylène-6 carbométhoxy-9 undécène-1 one-10 titrant 82 % en poids soit 131,4 m.moles. Le mélange réactionnel est chauffé à 60°C, puis on ajoute en 1 minute, 13,25 g de méthylbutynol (157,5 m.moles). Le mélange réactionnel est maintenu à 60° C sous agitation pendant 23 heures.

Après refroidissement à 20° C, le chlorhydrate de triéthylamine est séparé par filtration et est lavé avec 140 $cm^3$ d'acétone.

Le précipité sec pèse 13,63 g et contient 2,6 mg de cuivre. Le filtrat, après concentration à 40° C sous pression réduite (20 mm de mercure; 2,7 kPa), pèse 54,19 g et contient 0,488 g de cuivre.

Le taux de transformation des produits chlores mis en oeuvre est de 100 % et le rendement en produits de condensation (qui sont identiques à ceux obtenus dans l'exemple 25) est de 75,4 % par rapport aux produits chlorés mis en oeuvre. La sélectivité est de 95 %.

Le filtrat est extrait 2 fois successives par 200 $cm^3$ d''éther éthylique après décantation pendant 1 heure à chaque fois.

On obtient finalement un raffinat qui contient 14,18 g des produits de condensation et 0,475 g de cuivre et qui peut être utilisé directement dans une opération ultérieure.

On récupère ainsi 89,7 % du cuivre mis en oeuvre.

Les phases éthérées, après concentration, fournissent 39,86 g de produits de condensation.

**EXEMPLE 27**

Dans un ballon en verre de 50 $cm^3$ muni d'une agitation magnétique, on introduit successivement, sous atmosphère d'argon, 1,9 g du raffinat obtenu à l'exemple 26 contenant 1 milliatomegramme de cuivre, 10 $cm^3$ de triéthylamine (72,5 m.moles), 5,72 g d'un mélange 45/55 de chloro-3 diméthyl-2,6 carbométhoxy-9 undécadiène-1,6 one-10 et de chloro-3 méthyl-2 méthylène-6 carbométhoxy-9 undécène-1 one-10 titrant 82 % en poids, soit 16,37 m.moles, et 1,74 g de méthylbutynol (20,7 m.moles).

Le mélange réactionnel est agité à 60° C pendant 23 heures.

Après refroidissement à 20° C, on ajoute 20 $cm^3$ d'acétone puis on élimine le chlorhydrate de triéthylamine par filtration. Le précipité sec pèse 1,11 g.

Le filtrat, après concentration à 40° C sous pression réduite (20 mm de mercure; 2,7 kPa), pèse 8,5 g.

L'analyse du filtrat concentré montre que le taux de transformation des produits chlorés mis en oeuvre est de 39,6 % et que le rendement en produits de condensation est de 18,8 % par rapport aux produits chlorés mis en oeuvre. La sélectivité est de 95 %.

Après traitement du filtrat par l'éther éthylique dans les conditions décrites dans l'exemple 26, on obtient une phase éthérée qui fournit 3,72 g de produits de condensation.

**Revendications**

pour les Etats contractants BE, CH, DE, FR, GB, IT, LI, LU, NL, SE

1 - Un procédé de préparation de dérivés acétyléniques de formule générale:

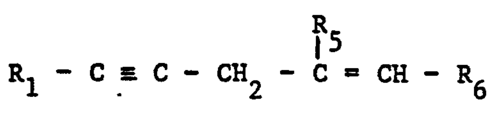

dans laquelle

- $R_1$ représente un atome d'hydrogène, un radical phényle ou un radical de formule générale:

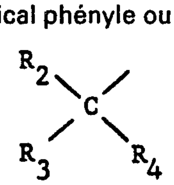

dans laquelle

$R_2$ et $R_3$, identiques ou différents, représentent un atome d'hydrogène ou un radical aliphatique saturé ou non saturé éventuellement substitué par un radical cycloaliphatique à 5 ou 6 chaînons contenant éventuellement une ou deux doubles liaisons et étant éventuellement substitué par un ou plusieurs radicaux

alcoyles contenant 1 à 4 atomes de carbone, l'un au moins des symboles $R_2$ et $R_3$ représentant un radical aliphatique saturé ou non saturé ou bien $R_2$ et $R_3$ forment ensemble un radical cycloaliphatique à 5 ou 6 chaînons contenant éventuellement une ou deux doubles liaisons et étant éventuellement substitué par un ou plusieurs radicaux alcoyles contenant 1 à 4 atomes de carbone,

$R_4$ représente un atome d'hydrogène ou un radical aliphatique saturé ou non saturé ou un radical hydroxy, alcoyloxy dont la partie alcoyle contient 1 à 4 atomes de carbone, alcoylcarbonyloxy dont la partie alcoyle contient 1 à 4 atomes de carbone, méthanesulfonyloxy, benzènesulfonyloxy ou p.toluènesulfonyloxy,

- $R_5$ représente um atome d'hydrogène ou un radical aliphatique saturé et

- $R_6$ représente un atome d'hydrogène ou un radical aliphatique saturé ou non saturé ou un radical acétyle, formyle éventuellement sous forme d'acétal, hydroxy, d'ester ou alcoyloxycarbonyle, étant entendu que les radicaux aliphatiques saturés contiennent 1 à 11 atomes de carbone et que les radicaux aliphatiques insaturés contiennent 2 à 11 atomes de carbone et une ou plusieurs doubles liaisons et sont éventuellement substitués par un ou plusieurs radicaux, identiques ou différents, choisis parmi les radicaux acétyle, formyle éventuellement sous forme d'acétal, hydroxy éventuellement sous forme d'éther ou d'ester ou alcoyloxycarbonyle par action d'un dérivé acétylénique de formule générale:

$R_1 - C \equiv C - H$

dans laquelle $R_1$ est défini comme précédemment, avec un dérivé allylique de formule générale:

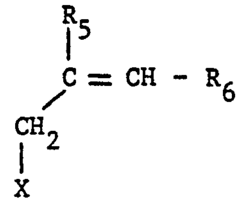

ou

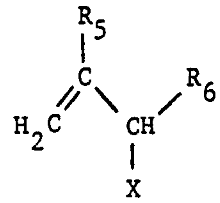

dans laquelle $R_5$ et $R_6$ sont définis comme précédemment et X représente um atome d'halogène ou un radical méthanesulfonyloxy, benzènesulfonyloxy, p.toluènesulfonyloxy ou ammonium quaternaire carsctérisé en ce que l'on opère en présence de 0,1 à 20 % en mole par rapport au dérivé allylique d'un dérivé du cuivre choisi parmi les sels cuivreux et les complexes du cuivre (1) ou les sels cuivriques ou les complexes du cuivre (II) préalablement réduits et de 1 à 20 moles par mole de dérivé allylique mis en oeuvre d'une base organique anhydre choisie parmi la triéthylsmine, la tributylsmine, la pyridine, la lutidine et la collidine à une température comprise entre 20 et 100° C.

2 - Procédé selon la revendication 1 caractérisé en ce que les sels cuivreux sont choisis parmi CuCl, $Cu_2O$, CuI, CuBr, CuCN, $Cu-C \equiv C-Cu$, $CuOCOCH_3$, $CuNO_3$, $C_6H_5-Cu$, $Cu(CH_3COCHCOCH_3)$, $(CH_3)_2C(OH)-C \equiv C-Cu$, les complexes du cuivre (I) sont choisis parmi [CuCl (cyclooctadiène-1,5$]_2$, $CuCl[N(C_2H_5)_3]_3$, $CuCl[N(C_2H_5)_3]_2$, $CuBr[N(C_2H_5)_3]_3$, $[CuCl(NC-R)]_n$, CuCl $(pyridine)_n$ ou CuCl (adiponitrile), les sels cuivriques sont choisis parmi $CuCl_2$, $CuBr_2$, $Cu(CN)_2$, $Cu(OCOCH_3)_2$ ou $Cu(NO_3)_2$ et les complexes du cuivre (II) sont choisis parmi $CuCl_2 (amine)_n$ ou $CuCl_2(CH_3CN)_2)$.

3 - Procédé selon la revendication 1 caractérisé en ce que l'on utilise 1 à 20 moles de dérivé acétylénique par mole de dérivé allylique mis en oeuvre.

4 - Procédé selon la revendication 1 caractérisé en ce que le catalyseur à base de cuivre (I) est utilisé en présence d'un co-catalyseur choisi parmi les halogénures d'ammonium, les halogénures de phosphonium, les halogénures de métal alcalin ou alcalino-terreux.

5 - Procédé selon la revendication 4 caractérisé en ce que le rapport molaire co-catalyseur/catalyseur est compris entre 1 et 50.

6 - Procédé selon la revendication 1 caractérisé en ce que, pour pouvoir recycler le catalyseur, on filtre le mélange réactionnel pour séparer l'halohydrate de la base organique puis on extrait le filtrat, une ou plusieurs fois, au moyen d'un solvant organique ne solubilisant pas les complexes de bases organiques avec le catalyseur au cuivre choisi parmi les hydrocarbures aliphatiques ou cycloaliphatiques et les éthers aliphatiques de façon à obtenir un extrait contenant les produits organiques résultant de la mise en oeuvre du procédé et un raffinat contenant le catalyseur qui peut être recyclé.

7 - Les nouveaux dérivés acétyléniques de formule générale:

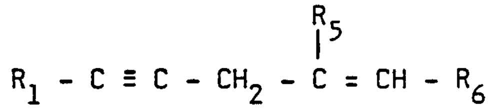

dans laquelle $R_1$ est défini comme dans la revendication 1, $R_5$ représente un radical aliphatique saturé contenant 1 à 11 atomes de carbone et $R_6$ représente un radical de formule générale:

$-CH_2CH_2 R_7$

dans laquelle $R_7$ représente un atome d'hydrogène ou un radical aliphatique saturé contenant 1 à 9 atomes de carbone ou un radical aliphatique insaturé contenant 2 à 9 atomes de carbone éventuellement substitué par

un ou plusieurs radicaux, identiques ou différents, choisis parmi les radicaux acétyle, formyle éventuellement sous forme d'acétal, hydroxy éventuellement sous forme d'éther ou d'ester ou alcoyloxycarbonyle, ou un radical acétyle, formyle éventuellement sous forme d'acétal ou un radical hydroxy éventuellement sous forme d'éther ou d'ester ou alcoyloxycarbonyle.

**Revendications**

pour l'Etat contractant AT

1 - Un procédé de préparation de dérivés acétyléniques de formule générale:

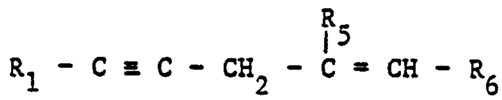

dans laquelle

- $R_1$ représente un atome d'hydrogène, un radical phényle ou un radical de formule générale:

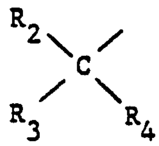

dans laquelle

$R_2$ et $R_3$, identiques ou différents, représentent un atome d'hydrogène ou un radical aliphatique saturé ou non saturé éventuellement substitué par un radical cycloaliphatique à 5 ou 6 chaînons contenant éventuellement une ou deux doubles liaisons et étant éventuellement substitué par un ou plusieurs radicaux alcoyles contenant 1 à 4 atomes de carbone, l'un au moins des symboles $R_2$ et $R_3$ représentant un radical aliphatique saturé ou non saturé ou bien $R_2$ et $R_3$ forment ensemble un radical cycloaliphatique à 5 ou 6 chaînons contenant éventuellement une ou deux doubles liaisons et étant éventuellement substitué par un ou plusieurs radicaux alcoyles contenant 1 à 4 atomes de carbone,

$R_4$ représente un atome d'hydrogène ou un radical aliphatique saturé ou non saturé ou un radical hydroxy, alcoyloxy dont la partie alcoyle contient 1 à 4 atomes de carbone, alcoylcarbonyloxy dont la partie alcoyle contient 1 à 4 atomes de carbone, méthanesulfonyloxy, benzènesulfonyloxy ou p.toluènesulfonyloxy,

$R_5$ représente un atome d'hydrogène ou un radical aliphatique saturé et

- $R_6$ représente un atome d'hydrogène ou un radical aliphatique saturé ou non saturé ou un radical acétyle, formyle éventuellement sous forme d'acétal, hydroxy éventuellement sous forme d'éther ou d'ester ou alcoyloxycarbonyle, étant entendu que les radicaux aliphatiques saturés contiennent 1 à 11 atomes de carbone et que les radicaux aliphatiques insaturés contiennent 2 à 11 atomes de carbone et une ou plusieurs doubles liaisons et sont éventuellement substitués par un ou plusieurs radicaux, identiques ou différents, choisis parmi les radicaux acétyle, formyle éventuellement sous forme d'acétal, hydroxy éventuellement sous forme d'éther ou d'ester ou alcoyloxycarbonyle par action d'un dérivé acétylénique de formule générale:

$R_1$- C ≡ C - H

dans laquelle $R_1$ est défini comme précédemment, avec un dérivé allylique de formule générale:

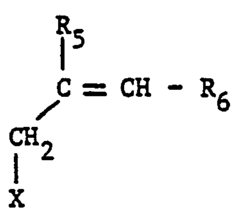

ou

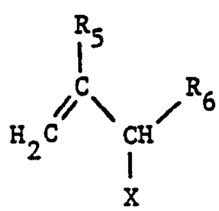

dans laquelle $R_5$ et $R_6$ sont définis comme précédemment et X représente un atome d'halogène ou un radical méthanesulfonyloxy, benzènesulfonyloxy, p.toluènesulfonyloxy ou ammonium quaternaire caractérisé en ce que l'on opère en présence de 0,1 à 20 % en mole par rapport au dérivé allylique d'un dérivé du cuivre choisi parmi les sels cuivreux et les complexes du cuivre (I) ou les sels cuivriques ou les complexes du cuivre (II) préalablement réduits et de 1 à 20 moles par mole de dérivé allylique mis en oeuvre d'une base organique anhydre choisie parmi la triéthylamine, la tributylamine, la pyridine, la lutidine et la collidine à une température comprise entre 20 et 100° C.

2 - Procédé selon la revendication 1 caractérisé en ce que les sels cuivreux sont choisis parmi CuCl, $Cu_2O$, CuI, CuBr, CuCN, Cu-C≡C-Cu, $CuOCOCH_3$, $CuNO_3$, $C_6H_5$-Cu, $Cu(CH_3COCHCOCH_3)$, $(CH_3)_2C(OH)$-C≡C-Cu, les

complexes du cuivre (I) sont choisis parmi [CuCl (cyclooctadiène-1,5]$_2$, CuCl[N($C_2H_5$)$_3$]$_3$, CuCl[N($C_2H_5$)$_3$]$_2$, CuBr[N($C_2H_5$)$_3$]$_3$, [CuCl(NC-R)]$_n$, CuCl (pyridine) $_n$ ou CuCl (adiponitrile), les sels cuivriques sont choisis parmi $CuCl_2$, $CuBr_2$, $Cu(CN)_2$, $Cu(OCOCH_3)_2$ ou $Cu(NO_3)_2$ et les complexes du cuivre (II) sont choisis parmi $CuCl_2$ (amine)$_n$ ou $CuCl_2(CH_3CN)_2$).

3 - Procédé selon la revendication 1 caractérisé en ce que l'on utilise 1 à 20 moles de dérivé acétylénique par mole de dérivé allylique mis en oeuvre.

4 - Procédé selon la revendication 1 caractérisé en ce que le catalyseur à base de cuivre (I) est utilisé en présence d'un co-catalyseur choisi parmi les halogénures d'ammonium, les halogénures de phosphonium, les halogénures de métal alcalin ou alcalino-terreux.

5 - Procédé selon la revendication 4 caractérisé en ce que le rapport molaire co-catalyseur/catalyseur eat compris entre 1 et 50.

6 - Procédé selon la revendication 1 caractérisé en ce que, pour pouvoir recycler le catalyseur, on filtre le mélange réactionnel pour séparer l'halohydrate de la base organique puis on extrait le filtrat, une ou plusieurs fois, au moyen d'un solvant organique ne solubilisant pas les complexes de bases organiques avec le catalyseur au cuivre choisi parmi les hydrocarbures aliphatiques ou cycloaliphatiques et les éthers aliphatiques de façon à obtenir un extrait contenant les produits organiques résultant de la mise en oeuvre du procédé et un raffinat contenant le catalyseur qui peut être recyclé.

## Patentansprüche

für die Vertragsstaaten BE, CH, DE, FR, GB, IT, LI, LU, NL, SE

1. Verfahren zur Herstellung von acetylenischen Derivaten der allgemeinen Formel:

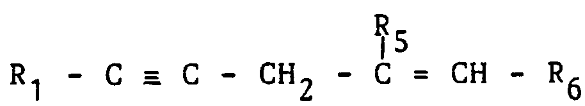

worin bedeuten:

- $R_1$ ein Halogenatom, einen Phenylrest oder einen Rest der allgemeinen Formel:

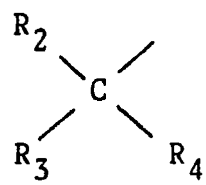

worin $R_2$ und $R_3$, die identisch oder verschieden sind, ein Wasserstoffatom oder einen gesättigten oder ungesättigten aliphatischen Rest bedeuten, der gegebenenfalls durch einen cycloaliphatischen Rest mit 5 oder 6 Kettengliedern, enthaltend gegebenenfalls 1 oder 2 Doppelbindungen und gegebenenfalls substituiert durch einen oder mehrere Alkylreste mit 1 bis 4 Kohlenstoffatomen substituiert ist, wobei wenigstens eines der Symbole $R_2$ und $R_3$ einen gesättigten oder ungesättigten aliphatischen Rest bedeutet, oder auch $R_2$ und $R_3$ bilden zusammen einen cycloaliphatischen Rest mit 5 oder 6 Kettengliedern, enthaltend gegebenenfalls 1 oder 2 Doppelbindungen, und gegebenenfalls substituiert durch einen oder mehrere Alkylreste mit 1 bis 4 Kohlenstoffatomen,

$R_4$ bedeutet ein Wasserstoffatom oder einen gesättigten oder ungesättigten aliphatischen Rest oder einen Hydroxyrest, Alkyloxy, dessen Alkylteil 1 bis 4 Kohlenstoffatome enthält, Alkylcarbonyloxy, dessen Alkylteil 1 bis 4 Kohlenstoffatome enthält, Methansulfonyloxy, Benzolsulfonyloxy oder p-Toluolsulfonyloxy,

-$R_5$ ein Wasserstoffatom oder einen gesättigten aliphatischen Rest und

-$R_6$ ein Wasserstoffatom oder einen gesättigten oder ungesättigten aliphatischen Rest oder einen Acetylrest, Formyl, gegebenenfalls in Acetalform, Hydroxy, gegebenenfalls in Ether- oder Esterform oder Alkyloxycarbonyl, wobei die gesättigten aliphatischen Reste 1 bis 11 Kohlenstoffatome enthalten, und die ungesättigten aliphatischen Reste 2 bis 11 Kohlenstoffatome und eine oder mehrere Doppelbindungen enthalten, und gegebenenfalls substituiert sind durch einen oder mehrere identische oder voneinander verschiedene Reste, ausgewählt unter den Resten Acetyl, Formyl, gegebenenfalls in Acetalform, Hydroxy, gegebenenfalls in Ether- oder Esterform oder Alkyloxycarbonyl, durch Einwirkung eines acetylenischen Derivats der allgemeinen Formel

$R_1 - C \equiv C - H$

worin $R_1$ wie vorstehend definiert ist, mit einem Allylderivat der allgemeinen Formel:

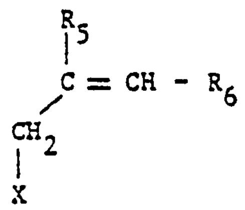

ou

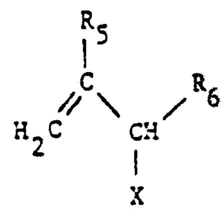

worin $R_5$ und $R_6$ wie vorstehend definiert sind und X ein Halogenatom oder einen Methansulfonyloxy-, Benzolsulfonyloxy-, p-Toluolsulfonyloxy- oder quaternären Ammoniumrest bedeuten, dadurch gekennzeichnet, daß man in Gegenwart von 0,1 bis 20 Mol-% in Bezug auf das Allylderivat eines Derivats des Kupfers, das ausgewählt ist unter den Cuprosalzen und den Komplexen des Kupfers (I) oder den Cuprisalzen oder den Komplexen des Kupfers (II), die vorher reduziert wurden, und von 1 bis 20 Mol je Mol eingesetztes Allylderivat einer wasserfreien organischen Base, ausgewählt unter Triethylamin, Tributylamin, Pyridin, Lutidin und Collidin,bei einer Temperatur zwischen 20 und 100°C arbeitet.

2. Verfahren gemäß Anspruch 1, dadurch gekennzeichnet, daß die Cuprosalze ausgewählt sind unter CuCl, $Cu_2O$, CuJ, CuBr, CuCN, $Cu-C\equiv C-Cu$, $CuOCOCH_3$, $CuNO_3$, $C_6H_5-Cu$, $Cu(CH_3COCHCOCH_3)$ $(CH_3)_2C(OH)-C=C-Cu$, die Komplexe des Kupfers (I) ausgewählt sind unter $[CuCl-(Cyclooctadien-1,5)]_2$, $CuCl[N(C_2H_5)_3]_3$, $CuCl[(C_2H_5)_3]_2$, $CuBr[N(C_2H_5)_3]_3$, $[CuCl(NC-R)]_n$, $CuCl-(Pyridin)_n$ oder CuCl-(Adiponitril), die Cuprisalze ausgewählt sind unter $CuCl_2$, $CuBr_2$, $Cu(CN)_2$, $Cu(OCOCH_3)_2$ oder $Cu(NO_3)_2$ und die Komplexe des Kupfers (II) ausgewählt sind unter $CuCl_2$-(Amin) oder $CuCl_2(CH_3CN)_2)$.

3. Verfahren gemäß Anspruch 1, dadurch gekennzeichnet, daß man 1 bis 20 Mol acetylenisches Derivat pro Mol eingesetztes Allylderivat verwendet.

4. Verfahren gemäß Anspruch 1, dadurch gekennzeichnet, daß der Katalysator auf Basis von Kupfer (I) in Gegenwart eines Cokatalysators, ausgewählt unter den Ammoniumhalogeniden, Phosphoniumhalogeniden, Alkalimetall- oder Erdalkalimetallhalogeniden, verwendet wird.

5. Verfahren gemäß Anspruch 4, dadurch gekennzeichnet, daß das Molverhältnis Cokatalysator/Katalysator zwischen 1 und 50 beträgt.

6. Verfahren gemäß Anspruch 1, dadurch gekennzeichnet, daß man, um den Katalysator recyclieren zu können, das Reaktionsgemisch filtriert, um das Hydrohalogenid der organischen Base abzutrennen, daß man das Filtrat dann ein- oder mehrmals mittels eines organischen Lösungsmittelsextrahiert, das die Komplexe der organischen Basen mit dem Kupferkatalysator nicht löst, ausgewählt unter den aliphatischen oder cycloaliphatischen Kohlenwasserstoffen und den aliphatischen Ethern, derart, daß man einen Extrakt erhält, der die organischen produkte, die sich von dem Einsatz des Verfahrens ergeben, und ein Raffinat, enthaltend den Katalysator, der recycliert werden kann, enthält.

7. Die neuen acetylenischen Derivate der allgemeinen Formel:

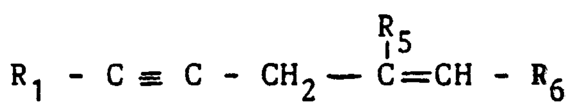

worin $R_1$ wie in Anspruch 1 definiert ist, $R_5$ einen gesättigten aliphatischen Rest mit 1 bis 11 Kohlenstoffatomen und $R_6$ einen Rest der allgemeinen Formel

$-CH_2CH_2 R_7$

bedeutet, worin $R_7$ ein Wasserstoffatom oder einen gesättigten aliphatischen Rest mit 1 bis 9 Kohlenstoffatomen oder einen ungesättigten aliphatischen Rest mit 2 bis 9 Kohlenstoffatomen bedeutet, gegebenenfalls substituiert durch einen oder mehrere identische oder verschiedene Reste, ausgewählt unter den Resten Acetyl, Formyl, gegebenenfalls in Acetalform, Hydroxy, gegebenenfalls in Ether- oder Esterform oder Alkyloxycarbonyl; oder einen Acetylrest, Formyl, gegebenenfalls in Acetalform oder einen Hydroxyrest, gegebenenfalls in Ether- oder Esterform, oder Alkoxycarbonylrest bedeutet.

**Patentansprüche**

für den Vertragsstaat AT:

1. Verfahren zur Herstellung von Acetylenderivaten der allgemeinen Formel:

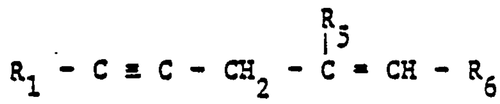

in welcher:

- $R_1$ bedeutet ein Wasserstoffatom, einen Phenylrest oder einen Rest der allgemeinen Formel:

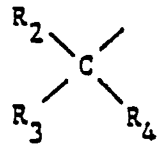

in welcher:

-$R_2$ und $R_3$, die gleich oder verschieden sind, bedeuten ein Wasserstoffatom oder einen gesättigten oder ungesättigten aliphatischen Rest, gegebenenfalls substituiert durch einen cycloaliphatischen Rest mit 5 oder 6 Gliedern, gegebenenfalls mit einer oder zwei Doppelbindungen und gegebenenfalls substituiert durch einen oder mehrere Alkylreste mit 1 bis 4 Kohlenstoffatomen, wobei mindestens eines der Symbole $R_2$ und $R_3$ einen gesättigten oder ungesättigten aliphatischen Rest bedeuten, oder $R_2$ und $R_3$ bilden zusammen einen cycloaliphatischen Rest mit 5 oder 6 Gliedern mit gegebenenfalls einer oder zwei Doppelbindungen und gegebenenfalls substituiert durch einen oder mehrere Alkylreste mit 1 bis 4 Kohlenstoffatomen,

$R_4$ bedeutet ein Wasserstoffatom oder einen gesättigten oder ungesättigten aliphatischen Rest oder einen Hydroxyrest, einen Alkoxyrest, dessen Alkylteil 1 bis 4 Kohlenstoffatome enthält, einen Alkylcarbonyloxyrest, dessen Alkylteil 1 bis 4 Kohlenstoffatome enthält, einen Methansulfonyloxyrest, einen Benzolsulfonyloxyrest oder einen p.Toluolsulfonyloxyrest,

- $R_5$ bedeutet ein Wasserstoffatom oder einen gesättigten aliphatischen Rest und

- $R_6$ bedeutet ein Wasserstoffatom oder einen gesättigten oder ungesättigten aliphatischen Rest oder einen Acetylrest, einen Formylrest, gegebenenfalls in Form des Acetyls einen Hydroxyrest, gegebenenfalls in Form des Äthers oder Esters, oder einen Alkoxycarbonylrest, wobei selbstverständlich die gesättigten aliphatischen Reste 1 bis 11 Kohlenstoffatome enthalten und die ungesättigten aliphatischen Rest 2 bis 11 Kohlenstoffatome enthalten und eine oder mehrere Doppelbindungen aufweisen und gegebenenfalls durch einen oder mehrere Reste substituiert sind, die gleich oder verschieden sind und ausgewählt sind aus dem Acetylrest, dem Formylrest, gegebenenfalls in Form des Acetals, dem Hydroxyrest, gegebenenfalls in Form des Äthers oder Esters, oder dem Alkoxycarbonylrest, durch Umsetzung eines Acetylenderivats der allgemeinen Formel:

$R_1 - C \equiv C - H$,

in welcher $R_1$ wie oben definiert ist, mit einem Allylderivat der allgemeinen Formel:

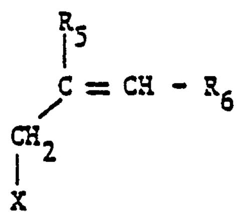 oder 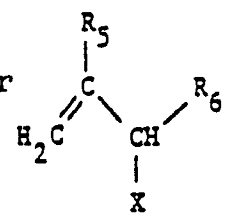

in welcher $R_5$ und $R_6$ wie oben definiert sind und X ein Halogenatom oder einen Methansulfonyloxy-, Benzolsulfonyloxy-, p.Toluolsulfonyloxyrest oder quaternäres Ammonium bedeutet, dadurch gekennzeichnet, daß man in Gegenwart von 0,1 bis 20 mol/% bezogen auf das Allylderivat eines Kupferderivates, ausgewählt aus den Kupfer(I)-salzen und den Kupfer(I)-komplexen oder den vorher reduzierten Kupfer(II)-salzen oder Kupfer(II)-komplexen, und von 1 bis 20 mol pro Mol eingesetzten Allylderivat einer wasserfreien organischen Base, ausgewählt aus dem Triethylamin, dem Tributylamin, dem Pyridin, dem Lutidin und dem Collidin, bei einer Temperatur zwischen 20 und 100° C arbeitet.

2. Verfahren nach Anspruch 1, dadurch gekennzeichnet, daß die Kupfer(I)-salze ausgewählt sind aus CuCl, $Cu_2O$, CuJ, CuBr, CuCN, $Cu-C \equiv C-Cu$, $CuOCOCH_3$, $CuNO_3$, $C_6H_5-Cu$, $Cu(CH_3COCHCOCH_3)$, $(CH_3)_2C(OH)-C \equiv C-Cu$, die Kupfer(I)-komplexe ausgewählt sind aus $[CuCl\ (1{,}5\text{-cyclooctadien})]_2$ $CuCl[N(C_2H_5)_3]_3$, $CuCl[N(C_2H_5)_3]_2$, $CuBr[N(C_2H_5)_3]_3$ $[CuCl(NC\text{-}R)]_n$ CuCl (pyridin) oder CuCl (adiponitril) die Kupfer(II)-salze ausgewählt sind aus $CuCl_2$ $CuBr_2$ $Cu(CN)_2$ $Cu(OCOCH_3)_2$ oder $Cu(NO_3)_2$ und die Kupfer(II)-komplexe ausgewählt sind aus $CuCl_2$ $(amin)_n$ oder $CuCl_2(CH_3CN)_2$).

3. Verfahren nach Anspruch 1, dadurch gekennzeichnet, daß man 1 bis 20 mol Acetylenderivat pro Mol eingesetztem Allylderivat verwendet.

4. Verfahren nach Anspruch 1, dadurch gekennzeichnet, daß der Katalysator auf Basis von Kupfer (I) in Gegenwart eines Cokatalysators, ausgewählt aus den Ammoniumhalogeniden, den Phosphoniumhalogeniden, den Alkali- oder Erdalkalimetallhalogeniden verwendet wird.

5. Verfahren nach Anspruch 4, dadurch gekennzeichnet, daß das molare Verhältnis Cokatalysator/Katalysator zwischen 1 und 50 ist.

6. Verfahren nach Anspruch 1, dadurch gekennzeichnet, daß man zur Rückgewinnung des Katalysators die Reaktionsmischung zur Abtrennung des Halohydrates der organischen Base filtriert und dann das Filtrat ein oder mehrere Male mittels eines organischen, nicht die Komplexe von organischen Basen mit dem Kupferkatalysator solubilisierenden Lösungsmittels, ausgewählt aus den aliphatischen oder cycloaliphatischen Kohlenwasserstoffen und den aliphatischen Äthern, derart extrahiert, daß man ein Extrakt erhält, das die aus dem Verfahren erhaltenen, organischen Verbindungen und ein Raffinat enthält, das den rückgewinnbaren Katalysator enthält.

**Claims**

for the contracting States BE CH DE FR GB IT LI LU NL SE

1. A process for the preparation of acetylene derivatives of general formula:

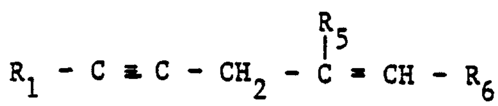

in which:

- $R_1$ denotes a hydrogen atom, a phenyl radical or a radical of general formula:

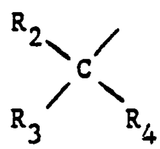

in which:

- $R_2$ and $R_3$, which are identical or different, denote a hydrogen atom or a saturated or unsaturated aliphatic radical substituted if desired by a 5- or 6-membered alicyclic radical containing, if desired, one or two double bonds and being substituted, if desired, by one or more alkyl radicals containing 1 to 4 carbon atoms, at least one of the symbols $R_2$ and $R_3$ denoting a saturated or unsaturated aliphatic radical or else $R_2$ and $R_3$ together form a 5- or 6-membered alicyclic radical containing, if desired, one or two double bonds and being substituted, if desired, by one or more alkyl radicals containing 1 to 4 carbon atoms,

- $R_4$ denotes a hydrogen atom or a saturated or unsaturated aliphatic radical or a hydroxy radical, an alkoxy radical in which the alkyl part contains 1 to 4 carbon atoms, an alkylcarbonyloxy radical in which the alkyl part contains 1 to 4 carbon atoms, or a methanesulphonyloxy, benzenesulphonyloxy or p-toluenesulphonyloxy radical,

- $R_5$ denotes a hydrogen atom or a saturated aliphatic radical and

- $R_6$ denotes a hydrogen atom or a saturated or unsaturated aliphatic radical or an acetyl radical, a formyl radical, if desired in acetal form, a hydroxy radical, if desired in ether or ester form, or an alkoxycarbonyl radical, it being understood that the saturated aliphatic radicals contain 1 to 11 carbon atoms and that the unsaturated aliphatic radicals contain 2 to 11 carbon atoms and one or more double bonds and are substituted, if desired, by one or more identical or different radicals chosen from acetyl radicals, formyl radicals, if desired in acetal form, hydroxy radicals, if desired in ether or ester form, or alkoxycarbonyl radicals by the reaction of an acetylene derivative of general formula:

$R_1$- C ≡ C - H

in which $R_1$ is defined as above, with an allyl derivative of general formula:

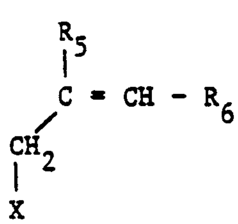

or

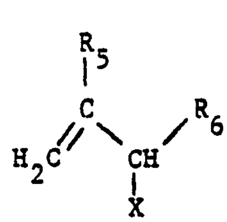

in which $R_5$ and $R_6$ are defined as above and X denotes a halogen atom or a methanesulphonyloxy, benzenesulphonyloxy or p-toluenesulphonyloxy radical or quaternary ammonium, characterized in that the reaction is carried out in the presence of 0.1 to 20% on a molar basis relative to the allyl derivative of a copper derivative chosen from cuprous salts and copper (I) complexes or cupric salts or copper (II) complexes which

are reduced beforehand and of 1 to 20 moles per mole of allyl derivative employed of an anhydrous organic base chosen from triethylamine, tributylamine, pyridine, lutidine and collidine at a temperature of between 20 and 100°C.

2. Process according to Claim 1, characterized in that the cuprous salts are chosen from CuCl, $Cu_2O$, CuI, CuBr, CuCN, Cu-C≡C-Cu, $CuOCOCH_3$, $CuNO_3$, $C_6H_5$-Cu, $Cu(CH_3COCHCOCH_3)$, $(CH_3)_2C(OH)$-C≡C-Cu, the copper (I) complexes are chosen from $[CuCl(1,5\text{-cyclooctadiene})]_2$, $CuCl[N(C_2H_5)_3]_3$ $CuCl[N(C_2H_5)_3]_2$, $CuBr[N(C_2H_5)_3]_3$, $[CuCl(NC\text{-}R)]_n$, $CuCl(pyridine)_n$ or CuCl(adiponitrile), the cupric salts are chosen from $CuCl_2$, $CuBr_2$, $Cu(CN)_2$, $Cu(OCOCH_3)_2$ or $Cu(NO_3)_2$ and the copper(II) complexes are chosen from $CuCl_2(amine)_n$ or $CuCl_2(CH_3CN)_2$.

3. Process according to Claim 1, characterized in that 1 to 20 moles of acetylene derivative are used per mole of allyl derivative employed.

4. Process according to Claim 1, characterized in that the catalyst based on copper(I) is used in the presence of a cocatalyst chosen from ammonium halides, phosphonium halides and alkali metal or alkaline-earth metal halides.

5. Process according to Claim 4, characterized in that the cocatalyst/catalyst molar ratio is between 1 and 50.

6. Process according to Claim 1, characterized in that, to enable the catalyst to be recycled, the reaction mixture is filtered to separate off the hydrohalide of the organic base and then the filtrate is extracted one or more times by means of an organic solvent which does not dissolve the complexes of organic bases with the copper catalyst, which solvent is chosen from aliphatic or alicyclic hydrocarbons and aliphatic ethers so as to produce an extract containing the organic products resulting from the implementation of the process and a raffinate containing the catalyst which may be recycled.

7. The new acetylene derivatives of general formula:

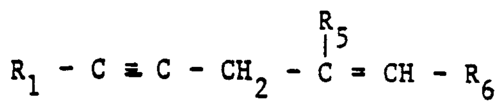

in which $R_1$ is defined as in Claim 1, $R_5$ denotes a saturated aliphatic radical containing 1 to 11 carbon atoms and $R_6$ denotes a radical of general formula:

$-CH_2CH_2\,R_7$

in which $R_7$ denotes a hydrogen atom or a saturated aliphatic radical containing 1 to 9 carbon atoms or an unsaturated aliphatic radical containing 2 to 9 carbon atoms substituted, if desired, by one or more identical or different radicals chosen from acetyl radicals, formyl radicals, if desired in acetal form, hydroxy radicals, if desired in ether or ester form or an alkoxycarbonyl radical, or an acetyl radical, a formyl radical, if desired in acetal form, or a hydroxy radical, if desired in ether or ester form or an alkoxycarbonyl radical.

## Claims

for the contracting States AT

1. A process for the preparation of acetylene derivatives of general formula:

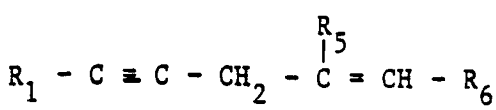

in which:

- $R_1$ denotes a hydrogen atom, a phenyl radical or a radical of general formula:

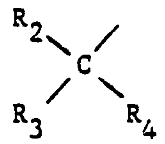

in which:

- $R_2$ and $R_3$, which are identical or different, denote a hydrogen atom or a saturated or unsaturated aliphatic radical substituted, if desired, by a 5- or 6-membered alicyclic radical containing, if desired, one or two double bonds and being substituted, if desired, by one or more alkyl radicals containing 1 to 4 carbon atoms, at least one of the symbols $R_2$ and $R_3$ denoting a saturated or unsaturated aliphatic radical or else $R_2$ and $R_3$ together form a 5- or 6-membered alicyclic radical containing, if desired, one or two double bonds and being substituted, if desired, by one or more alkyl radicals containing 1 to 4 carbon atoms,
- $R_4$ denotes a hydrogen atom or a saturated or unsaturated aliphatic radical or a hydroxy radical, an alkoxy

radical in which the alkyl part contains 1 to 4 carbon atoms, an alkylcarbonyloxy radical in which the alkyl part contains 1 to 4 carbon atoms, or a methanesulphonyloxy, benzenesulphonyloxy or p-toluenesulphonyloxy radical,

- $R_5$ denotes a hydrogen atom or a saturated aliphatic radical and
- $R_6$ denotes a hydrogen atom or a saturated or unsaturated aliphatic radical or an acetyl radical, a formyl radical, if desired in acetal form, a hydroxy radical, if desired in ether or ester form, or an alkoxycarbonyl radical, it being understood that the saturated aliphatic radicals contain 1 to 11 carbon atoms and that the unsaturated aliphatic radicals contain 2 to 11 carbon atoms and one or more double bonds and are substituted, if desired, by one or more identical or different radicals chosen from acetyl radicals, formyl radicals, if desired in acetal form, hydroxy radicals, if desired in ether or ester form, or alkoxycarbonyl radicals by the reaction of an acetylene derivative of general formula:

$R_1$- C ≡ C - H

in which $R_1$ is defined as above, with an allyl derivative of general formula:

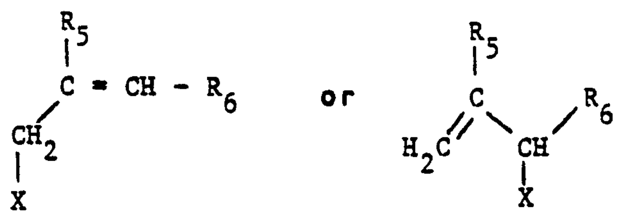

in which $R_5$ and $R_6$ are defined as above and X denotes a halogen atom or a methanesulphonyloxy, benzenesulphonyloxy or p-toluenesulphonyloxy radical or quaternary ammonium, characterized in that the reaction is carried out in the presence of 0.1 to 20% on a molar basis relative to the allyl derivative of a copper derivative chosen from cuprous salts and copper(I) complexes or cupric salts or copper (II) complexes which are reduced beforehand and of 1 to 20 moles per mole of allyl derivative employed of an anhydrous organic base chosen from triethylamine, tributylamine, pyridine, lutidine and collidine at a temperature of between 20 and 100°C.

2. Process according to Claim 1, characterized in that the cuprous salts are chosen from CuCl, $Cu_2O$, CuI, CuBr, CuCN, Cu-C≡C-Cu, $CuOCOCH_3$, $CuNO_3$, $C_6H_5$-Cu, $Cu(CH_3COCHCOCH_3)$, $(CH_3)_2C(OH)$-C≡C-Cu, the copper(I) complexes are chosen from $[CuCl(1,5\text{-cyclooctadiene})]_2$, $CuCl[N(C_2H_5)_3]_3$ $CuCl[N(C_2H_5)_3]_2$, $CuBr[N(C_2H_5)_3]_3$, $[CuCl(NC\text{-}R)]_n$, $CuCl(pyridine)_n$ or CuCl(adiponitrile), the cupric salts are chosen from $CuCl_2$, $CuBr_2$, $Cu(CN)_2$, $Cu(OCOCH_3)_2$ or $Cu(NO_3)_2$ and the copper(II) complexes are chosen from $CuCl_2(amine)_n$ or $CuCl_2(CH_3CN)_2$.

3. Process according to Claim 1, characterized in that 1 to 20 moles of acetylene derivative are used per mole of allyl derivative employed.

4. Process according to Claim 1, characterized in that the catalyst based on copper(I) is used in the presence of a cocatalyst chosen from ammonium halides, phosphonium halides and alkali metal or alkaline-earth metal halides.

5. Process according to Claim 4, characterized in that the cocatalyst/catalyst molar ratio is between 1 and 50.

6. Process according to Claim 1, characterized that, to enable the catalyst to be recycled, the reaction mixture is filtered to separate off the hydrohalide of the organic base and then the filtrate is extracted one or more times by means of an organic solvent which does not dissolve the complexes of organic bases with the copper catalyst, which solvent is chosen from aliphatic or alicyclic hydrocarbons and aliphatic ethers so as to produce an extract containing the organic products resulting from the implementation of the process and a raffinate containing the catalyst which may be recycled.